# DEMANDE DE BREVET EUROPEEN

(11) **EP 1 681 354 A2**
(43) Date de publication de la demande: **19.07.2006**
(21) Numéro de dépôt: 06004834.5
(22) Date de dépôt: 21.03.1995
(51) Int. Cl.: C12N 15/861, A61K 48/00, C12N 15/60, C12N 15/12, C12N 7/01, C12N 5/10, A61K 39/235, C12N 9/88, C12N 15/867, C12N 15/869, A61P 27/00

(54) **Virus recombinants codant pour une activité glutamate décarboxylase (GAD)**

(30) Priorité: 23.03.1994 FR 9403411; 09.11.1994 FR 9413487
(62) Demande divisionnaire de: 95914389.2
(71) Demandeur: Aventis Pharma S.A., 92160 Antony (FR)
(72) Inventeur: Bemelmans, Alexis, 75116 Paris (FR); Geoffroy, Marie-Claude, 78000 Versailles (FR); Horellou, Philippe, 75014 Paris (FR); Julien, Jean-François, 92160 Antony (FR); Mallet, Jacques, 75013 Paris (FR); Perricaudet, Michel, 28320 Ecrosnes (FR); Robert, Jean-Jacques, 92330 Sceaux (FR); Vigne, Emmanuelle, 94200 Ivry Sur Seine (FR)
(74) Mandataire: Chajmowicz, Marion

(57) **Abrégé**

La présente invention concerne des virus recombinants comportant une séquence d'ADN hétérologue codant pour une protéine ayant une activité glutamate décarboxylase (GAD), leur préparation, et leur utilisation thérapeutique, notamment pour le traitement et/ou la prévention des maladies neurodégénératives.

## Description

La présente invention concerne des vecteurs recombinants d'origine virale, leur préparation et leur utilisation, notamment pour le traitement et/ou la prévention des maladies neurodégénératives. Plus particulièrement, elle concerne des virus recombinants comportant une séquence d'ADN codant pour une protéine ayant une activité glutamate décarboxylase (GAD). L'invention concerne également la préparation de ces vecteurs, les compositions pharmaceutiques les contenant et leur utilisation thérapeutique, notamment en thérapie génique.

Les voies gabaergiques représentent le principal ensemble inhibiteur du système nerveux des vertébrés. L'altération de l'activité des neurones GABAergiques se manifeste immédiatement au niveau de l'organisme tout entier par des dyskinésies ou des convulsions. De plus, le rôle de l'acide gamma-amino-butyrique (GABA) cérébral ne se limite pas à la neurotransmission. Une action neurotrophique durant le développement lui a en effet été attribuée, en particulier dans la neurorétine. Par ailleurs, le GABA est également présent dans les cellules B des ilôts de Langerhans, où il semble jouer un rôle sur la régulation de la production d'insuline.

La possibilité de restaurer ou d'instaurer de novo, une synthèse GABA dans une région précise de l'organisme, présente donc un intérêt thérapeutique important, aussi bien pour des affections directement liées à la dégénérescence de neurones GABAergiques que pour celles qui répondent aux agonistes GABA. La présente invention apporte une solution particulièrement avantageuse à ce problème. La présente invention décrit en effet la mise au point de vecteurs, d'origine virale, utilisables en thérapie génique, capables d'exprimer in vivo de manière localisée et efficace une activité glutamate décarboxylase. La présente invention décrit ainsi une approche nouvelle pour traiter les affections liées à une déficience en GABA, consistant à induire la synthèse in vivo de GABA par libération ciblée d'un enzyme biologiquement actif.

L'activité glutamate décarboxylase (GAD) est un enzyme qui catalyse, de manière assez spécifique, la transformation du glutamate en acide gamma-amino-butyrique (GABA) à l'aide d'un cofacteur, le phosphate de pyridoxal (vitamine B6). A l'état natif, cet enzyme se présente sous forme d'un dimère de 120 kilodaltons. Après réduction des ponts disulfures, l'examen par électroimmunoanalyse (Western blot) spécifique révèle deux bandes à 65 et 67 kilodalton. Il a été récemment démontré que ces deux monomères correspondent à deux protéines différentes codées par des gènes distincts (Erlander et al., Neuron 7 (1991) 91). Les deux molécules, désormais appelées GAD 67 et GAD 65, se distinguent d'un point de vue enzymatique, la forme courte présentant une affinité plus réduite pour le phosphate de pyridoxal, et par leur localisation subcellulaire, la forme courte étant mieux représentée dans les prolongements neuronaux alors que la forme longue s'accumule au niveau du périkarya. La forme longue, de par sa dépendance plus réduite vis-à-vis de la concentration en phosphate de pyridoxal, est donc la plus à même de s'exprimer dans différents types cellulaires. La GAD 65, en revanche, présente la particularité de s'inactiver rapidement en l'absence de phosphate de pyridoxal en se transformant en apoenzyme, dénuée d'activité catalytique. La transformation inverse, d'apoenzyme en holoenzyme active, est relativement lente.

En outre, l'expression des GAD 67 et 65 n'est pas restreinte au tissu nerveux. Les deux GAD se rencontrent également dans les cellules β des îlots de Langerhans du pancréas. Des autoanticorps anti-GAD ont ainsi été détectés chez des patients atteints d'une maladie neurologique très rare, le Stiff-man syndrome, caractérisée par une hypertonie musculaire et accompagnée d'une dégénérescence de certains neurones GABAergiques. Une proportion non négligeable de ces malades (20 %) développe en outre un diabète de type I. De même, la GAD 67 est également relativement abondante dans le manchon du flagelle des spermatozoïdes où tout laisse à penser qu'elle joue un rôle important dans le catabolisme oxydatif.

La présente invention réside dans la découverte qu'il est possible de produire in vivo une activité enzymatique capable à son tour de favoriser ou d'induire la synthèse d'un neurotransmetteur, le GABA. La présente invention réside également dans la mise au point de vecteurs permettant la libération efficace, localisée et durable d'un enzyme actif in vivo sur la synthèse de GABA. La présente invention offre ainsi une nouvelle approche de thérapie génique particulièrement avantageuse pour le traitement et/ou la prévention des pathologies neurodégénératives.

La demanderesse s'est intéressée plus particulièrement à l'utilisation de vecteurs d'origine virale pour le transfert in vivo, notamment dans le système nerveux, d'une activité glutamate décarboxylase. De manière particulièrement avantageuse, la demanderesse a maintenant montré qu'il est possible de construire des virus recombinants contenant une séquence d'ADN codant pour la GAD, d'administrer ces virus recombinants in vivo, et que cette administration permet une expression stable, localisée et efficace d'une GAD biologiquement active in vivo, en particulier dans le système nerveux, et sans effet cytopathologique. La présente invention découle plus particulièrement de la mise en évidence de propriétés particulièrement avantageuses de certains virus pour l'expression d'une activité GAD, et de la construction de vecteurs viraux (virus défectifs, délétés de certaines régions virales, contenant certains promoteurs, etc) permettant une expression particulièrement efficace de l'activité GAD, et dans les tissus appropriés. La présente invention fournit ainsi des vecteurs viraux utilisables directement en thérapie génique, particulièrement adaptés et efficaces pour diriger l'expression de la GAD in vivo.

Un premier objet de l'invention réside donc dans un virus recombinant défectif comprenant une séquence d'ADN codant pour une protéine ayant une activité glutamate décarboxylase.

L'invention a également pour objet l'utilisation d'un tel virus recombinant défectif pour la préparation d'une composition pharmaceutique destinée au traitement et/ou à la prévention des maladies neurodégénératives.

Au sens de l'invention, le terme protéine ayant une activité glutamate décarboxylase (GAD) désigne toute protéine capable d'induire ou de favoriser la production de GABA à partir de glutamate. Plus particulièrement, la protéine ayant une activité glutamate décarboxylase est choisie parmi tout ou partie des protéines GAD65 et GAD67, ou de dérivés de celles-ci.

L'activité glutamate décarboxylase produite dans le cadre de la présente invention peut être une GAD humaine ou une GAD animale. La séquence d'ADN codant pour les GAD65 et 67 a été clonée et séquencée à partir de différentes espèces, et notamment à partir de l'homme (Bu et al., PNAS 89 (1992) 2115) et du rat (Julien et al., Neurosci. Letters 73 (1987) 173). Pour permettre leur incorporation dans un vecteur viral selon l'invention, ces séquences sont avantageusement modifiées, par exemple par mutagénèse dirigée, en particulier pour l'insertion de sites de restriction appropriés. Les séquences décrites dans l'art antérieur ne sont en effet pas construites pour une utilisation selon l'invention, et des adaptations préalables peuvent s'avérer nécessaires, pour obtenir des expressions importantes. Dans le cadre de la présente invention, on préfère utiliser une séquence d'ADN codant pour une GAD humaine. Par ailleurs, comme indiqué ci-avant, il est également possible d'utiliser une construction codant pour un dérivé de la GAD 65 ou 67, en particulier un dérivé des GAD humaines. Un tel dérivé comprend par exemple toute séquence obtenue par mutation, délétion et/ou addition par rapport à la séquence native, et codant pour un produit conservant la capacité d'induire ou de favoriser la production de GABA à partir de glutamate. Ces modifications peuvent être réalisées par les techniques connues de l'homme du métier (voir techniques générales de biologie moléculaire ci-après). L'activité biologique des dérivés ainsi obtenus peut ensuite être aisément déterminée, comme indiqué notamment dans l'exemple 2. Les dérivés au sens de l'invention peuvent également être obtenus par hybridation à partir de banques d'acides nucléiques, en utilisant comme sonde la séquence native ou un fragment de celle-ci.

Ces dérivés sont notamment des molécules ayant une plus grande affinité pour leurs sites de fixation, des molécules présentant une plus grande résistance aux protéases, des molécules ayant une efficacité thérapeutique plus grande ou des effets secondaires moindres, ou éventuellement de nouvelles propriétés biologiques. Les dérivés incluent également les séquences d'ADN modifiées permettant une expression améliorée in vivo.

Parmi les dérivés préférés, on peut citer plus particulièrement les variants naturels, les molécules dans lesquelles certains sites de N- ou O-glycosylation ont été modifiés ou supprimés, les molécules dans lesquelles un ou plusieurs résidus ont été substitués, ou les molécules dans lesquelles tous les résidus cystéine ont été substitués (mutéines). On peut citer également les dérivés obtenus par délétion de régions n'intervenant pas ou peu dans l'interaction avec les sites de liaison considérés ou exprimant une activité indésirable, et les dérivés comportant par rapport à la séquence native des résidus supplémentaires, tels que par exemple une méthionine N-terminale et/ou un signal de sécrétion et/ou un peptide de jonction.

La séquence d'ADN codant pour la GAD utilisée dans le cadre de la présente invention peut être un ADNc, un ADN génomique, ou une construction hybride consistant par exemple en un ADNc dans lequel seraient insérés un ou plusieurs introns. Il peut également s'agir de séquences synthétiques ou semisynthétiques. De manière particulièrement avantageuse, on utilise un ADNc ou un ADNg. En particulier, l'utilisation d'un ADN génomique permet une meilleure expression dans les cellules humaines.

Dans un premier mode de réalisation, la présente invention concerne un virus recombinant défectif comprenant une séquence d'ADNc codant pour une protéine ayant une activité GAD. Dans un autre mode de réalisation préféré de l'invention, le virus comprend une séquence d'ADNg codant pour une protéine ayant une activité GAD.

Les vecteurs de l'invention peuvent être préparés à partir de différents types de virus. Préférentiellement, on utilise des vecteurs dérivés des adénovirus, des virus adéno-associés (AAV), des virus de l'herpès (HSV) ou des rétrovirus.

Les virus selon l'invention sont défectifs, c'est-à-dire qu'ils sont incapables de se répliquer de façon autonome dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence d'ADN codant pour la GAD. Préférentiellement, le virus défectif conserve néanmoins les séquences de son génome qui sont nécessaires à l'encapsidation des particules virales.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande FR 93 05954). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprenent les ITR, une séquence permettant l'encapsidation et la séquence codant pour la protéine ayant une activité GAD. Encore plus préférentiellement, dans le génome des adénovirus de l'invention, le gène E1 et au moins l'un des gènes E2, E4, L1-L5 sont non fonctionnels. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN codant pour la GAD. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). Des stratégies de construction de vecteurs dérivés des adénovirus ont également été décrites dans les demandes n° FR 93 05954 et FR 93 08596.

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

Concernant les virus adéno-associés (AAV), il- s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant la séquence codant pour l'inhibiteur des ETS bordé de deux régions répétées inversées (ITR) d'AAV, et d'un plasmide portant les gènes d'encapsidation (gènes rep et cap) d'AAV. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, Bio Technology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, poi et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants comportant une séquence d'intérêt, un plasmide comportant notamment les LTR, la séquence d'encapsidation et ladite séquence d'intérêt est généralement construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques. Dans le cas particulier de la présente invention, les rétrovirus peuvent être préparés par recombinaison homologue entre un plasmide rétroviral contenant le gène codant pour la GAD et une lignée cellulaire transcomplémentante qui va permettre de produire des particules virales dont le génome code pour le transgène. A titre d'exemple de lignée on peut notamment citer la lignée cellulaire Ψ-2 qui provient de la lignée NIH-3T3 (lignée fibroblastique de souris) par transfection par pMOV-Ψ⁻, un plasmide contenant le génome du virus de la leucémie murine de Moloney (MoMuLV) dont la séquence d'encapsidation "Ψ" est délétée (Mann *et al.,* Cell, 33, 153-159, 1983).

Pour la mise en oeuvre de la présente invention, il est tout particulièrement avantageux d'utiliser un adénovirus ou un rétrovirus recombinant défectif. Les résultats donnés ci-après démontrent en effet les propriétés particulièrement intéressantes des adénovirus et rétrovirus pour l'expression in vivo d'une protéine ayant une activité GAD. De plus, les vecteurs adénoviraux et rétrovirus selon l'invention présentent en outre des avantages importants, tels que notamment leur très haute efficacité d'infection des cellules nerveuses, permettant de réaliser des infections à partir de faibles volumes de suspension virale. De plus, l'infection par ces vecteurs viraux de l'invention est très localisée au site d'injection, ce qui évite les risques de diffusion aux cellules voisines.

Avantageusement, dans les vecteurs de l'invention, la séquence codant pour la protéine ayant une activité GAD est placée sous le contrôle de signaux permettant son expression dans les cellules nerveuses. Préférentiellement, il s'agit de signaux d'expression hétérologues, c'est-à-dire de signaux différents de ceux naturellement responsables de l'expression de la GAD. Il peut s'agir en particulier de séquences responsables de l'expression d'autres protéines, ou de séquences synthétiques. Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris du virus utilisé. A cet égard, on peut citer par exemple les promoteurs E1A, MLP, CMV, LTR-RSV, etc. En outre, ces séquences d'expression peuvent être modifiées par addition de séquences d'activation, de régulation, ou permettant une expression tissu-spécifique. Il peut en effet être particulièrement intéressant d'utiliser des signaux d'expression actifs spécifiquement ou majoritairement dans les cellules nerveuses, de manière à ce que la séquence d'ADN ne soit exprimée et ne produise son effet que lorsque le virus a effectivement infecté une cellule nerveuse. A cet égard, on peut citer par exemple les promoteurs de l'énolase neurone-spécifique (Forss-Petter et al., Neuron 5 (1990) 187), de la GFAP (Sarkar et al., J. Neurochem. 57 (1991) 675), etc.

Dans un mode particulier de réalisation, l'invention concerne un virus recombinant défectif comprenant une séquence d'ADNc codant pour une protéine ayant une activité GAD sous le contrôle du promoteur LTR-RSV.

Dans un autre mode de réalisation particulier, l'invention concerne un virus recombinant défectif comprenant une séquence d'ADNg codant pour une protéine ayant une activité GAD sous le contrôle du promoteur LTR-RSV.

La demanderesse a en effet montré que le promoteur LTR du virus du sarcome de rous (RSV) permettait une expression durable et importante de l'activité GAD dans les cellules du système nerveux humain, notamment central.

Toujours dans un mode préféré, l'invention concerne un virus recombinant défectif comprenant une séquence d'ADN codant pour une protéine ayant une activité GAD sous le contrôle d'un promoteur permettant une expression majoritaire dans le système nerveux.

L'expression est considérée comme majoritaire au sens de l'invention lorsque, même si une expression résiduelle est observée dans d'autres types cellulaires, les niveaux d'expression sont supérieurs dans les cellules nerveuses.

Comme indiqué ci-avant, la présente invention concerne également toute utilisation d'un virus tel que décrit ci-dessus pour la préparation d'une composition pharmaceutique destinée au traitement et/ou à la prévention des maladies neurodégénératives. Plus particulièrement, elle concerne toute utilisation de ces virus pour la préparation d'une composition pharmaceutique destinée au traitement et/ou à la prévention de la maladie de Parkinson, de la maladie d'Alzheimer, de la sclérose latérale amyotrophique (ALS), de la maladie d'Huntington, de l'épilepsie, de la dégénéréscence cérébrovasculaire, etc.

La présente invention concerne également une composition pharmaceutique comprenant un ou plusieurs virus recombinants défectifs tels que décrits précédemment. Ces compositions pharmaceutiques peuvent être formulées en vue d'administrations par voie topique, orale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe dans le système nerveux du patient. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. L'injection directe dans le système nerveux du patient est avantageuse car elle permet de concentrer l'effet thérapeutique au niveau des tissus affectés. L'injection directe dans le système nerveux central du patient est avantageusement réalisée au moyen d'un appareil d'injection stéréotaxique. L'emploi d'un tel appareil permet en effet de cibler avec une grande précision le site d'injection. Des injections localisées, telles que notamment des injections intranigrales, dans le lobe temporal, dans les foyers épileptiques ou encore dans l'épithélium intestinal peuvent être particulièrement avantageuses.

Les doses de virus recombinant défectif utilisées pour l'injection peuvent être adaptées en fonction de différents paramètres, et notamment en fonction du vecteur viral, du mode d'administration utilisé, de la pathologie concernée ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature. Concernant les rétrovirus, les compositions selon l'invention peuvent comporter directement les cellules productrices, en vue de leur implantation.

La libération de GABA obtenue par thérapie génique selon l'invention constitue une approche thérapeutique particulièrement efficace pour contrecarrer les phénomènes excitotoxiques responsables de maladies nerveuses dégénératives comme les scléroses amyélotrophiques.

Les vecteurs de l'invention présentent donc des propriétés anticonvulsivantes et neuroprotectrices. Parmi les applications thérapeutiques des vecteurs de l'invention, on peut citer en particulier le traitement de certaines formes d'épilepsies, notamment celles résistantes à tout traitement pharmacologique ou chirurgical, telles que les épilepsies rebelles du lobe temporal. Les vecteurs de l'invention peuvent également être utilisés pour le traitement des lésions cérébrales excitotoxiques d'origines diverses, notamment d'origine ischémique, ainsi que pour le traitement des traumatismes médullaires susceptibles d'engendrer des lésions excitotoxiques. Ils présentent également un intérêt thérapeutique important dans le traitement du diabète, notamment de type I, par exemple par l'induction d'une tolérance à la GAD chez des individus prédiabétiques, permettant ainsi d'éviter l'attaque auto-immune responsable des symptômes de cette maladie. A cet égard, une synthèse de GAD, massive mais bien localisée, dans l'intestin par exemple, présente aussi un intérêt thérapeutique certain dans les cas de diabètes déclarés, en déprimant la réponse auto-immunitaire cellulaire.

Un autre objet de l'invention concerne toute cellule de mammifère infectée par un ou plusieurs virus recombinants défectifs tels que décrits ci-dessus. Plus particulièrement, l'invention concerne toute population de cellules humaines infectée par ces virus. Il peut s'agir en particulier de fibroblastes, myoblastes, hépatocytes, kératinocytes, cellules endothéliales, cellules gliales, etc.

Les cellules selon l'invention peuvent être issues de cultures primaires. Celles-ci peuvent être prélevées par toute technique connue de l'homme du métier, puis mises en culture dans des conditions permettant leur prolifération. S'agissant plus particulièrement de fibroblastes, ceux-ci peuvent être aisément obtenus à partir de biopsies, par exemple selon la technique décrite par Ham [Methods Cell.Biol. 21a (1980) 255]. Ces cellules peuvent être utilisées directement pour l'infection par les virus, ou conservées, par exemple par congélation, pour l'établissement de banques autologues, en vue d'une utilisation ultérieure. Les cellules selon l'invention peuvent également être des cultures secondaires, obtenues par exemple à partir de banques préétablies (voir par exemple EP228458, EP289034, EP400047, EP456640).

Les cellules en culture sont ensuite infectées par les virus recombinants, pour leur conférer la capacité de produire une activité GAD. L'infection est réalisée in vitro selon des techniques connues de l'homme du métier. En particulier, selon le type de cellules utilisé et le nombre de copies de virus par cellule désiré, l'homme du métier peut adapter la multiplicité d'infection et éventuellement le nombre de cycles d'infection réalisé. Il est bien entendu que ces étapes doivent être effectuées dans des conditions de stérilité appropriées lorsque les cellules sont destinées à une administration in vivo. Les doses de virus recombinant utilisées pour l'infection des cellules peuvent être adaptées par l'homme du métier selon le but recherché. Les conditions décrites ci-avant pour l'administration in vivo peuvent être appliquées à l'infection in vitro.

Un autre objet de l'invention concerne un implant comprenant des cellules mammifère infectées par un ou plusieurs virus recombinants défectifs telles que décrites ci-dessus, et une matrice extracellulaire. Préférentiellement, les implants selon l'invention comprennent 10⁵ à 10¹⁰ cellules. Plus préférentiellement, ils en comprennent 10⁶ à 10⁸.

Plus particulièrement, dans les implants de l'invention, la matrice extracellulaire comprend un composé gélifiant et éventuellement un support permettant l'ancrage des cellules.

Pour la préparation des implants selon l'invention, différents types de gélifiants peuvent être employés. Les gélifiants sont utilisés pour l'inclusion des cellules dans une matrice ayant la constitution d'un gel, et pour favoriser l'ancrage des cellules sur le support, le cas échéant. Différents agents d'adhésion cellulaire peuvent donc être utilisés comme gélifiants, tels que notamment le collagène, la gélatine, les glucosaminoglycans, la fibronectine, les lectines, etc. De préférence, on utilise dans le cadre de la présente invention du collagène. Il peut s'agir de collagène d'origine humaine, bovine ou murine. Plus préférenciellement, on utilise du collagène de type I.

Comme indiqué ci-avant, les compositions selon l'invention comprennent avantageusement un support permettant l'ancrage des cellules. Le terme ancrage désigne toute forme d'interaction biologique et/ou chimique et/ou physique entraînant l'adhésion et/ou la fixation des cellules sur le support. Par ailleurs, les cellules peuvent soit recouvrir le support utilisé, soit pénétrer à l'intérieur de ce support, soit les deux. On préfère utiliser dans le cadre de l'invention un support solide, non toxique et/ou bio-compatible. En particulier, on peut utiliser des fibres de polytétrafluoroéthylène (PTFE) ou un support d'origine biologique.

Les implants selon l'invention peuvent être implantés en différents sites de l'organisme. En particulier, l'implantation peut être effectuée au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse. Les implants selon l'invention sont particulièrement avantageux en ce sens qu'ils permettent de contrôler la libération du produit thérapeutique dans l'organisme : Celle-ci est tout d'abord déterminée par la multiplicité d'infection et par le nombre de cellules implantées. Ensuite, la libération peut être contrôlée soit par le retrait de l'implant, ce qui arrête définitivement le traitement, soit par l'utilisation de systèmes d'expression régulables, permettant d'induire ou de réprimer l'expression des gènes thérapeutiques.

La présente invention offre ainsi un moyen très efficace pour le traitement ou la prévention des maladies neurodégénératives. Elle est tout particulièrement adaptée au traitement des maladies d'Alzheimer, de Parkinson, de Huntington, de l'epilepsie et de l'ALS. En outre, ce traitement peut concerner aussi bien l'homme que tout animal tel que les ovins, les bovins, les animaux domestiques (chiens, chats, etc), les chevaux, les poissons, etc.

La présente invention sera plus complètement décrite à l'aide des exemples et figures qui suivent, qui doivent être considérés comme illustratifs et non limitatifs.

### Légende des figures

Figure 1 : Structure du vecteur pLTRIXGAD67
Figures 2a et 2b: Infection de lignées cellulaires HiB5 et ST14a par un clone Ψ-2 producteur de rétrovirus recombinants codant pour la GAD.
Figure 3: Activité glutamate décarboxylase de différents clones issus de lignées cellulaires infectées par un rétrovirus recombinant codant pour la GAD.
Figures 4 et 5: contrôle de la transplantation in vivo des clones HiB5 (figure 4B) et St14a (figures 5A et 5B) exprimant la GAD par rapport à des cellules ST14a non infectées (figure 4A) .
Figure 6: Libération de GABA in vitro par les cellules génétiquement modifiées.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la precipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodêoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B, et Faloona F.A., Meth. Enzym. 155. (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

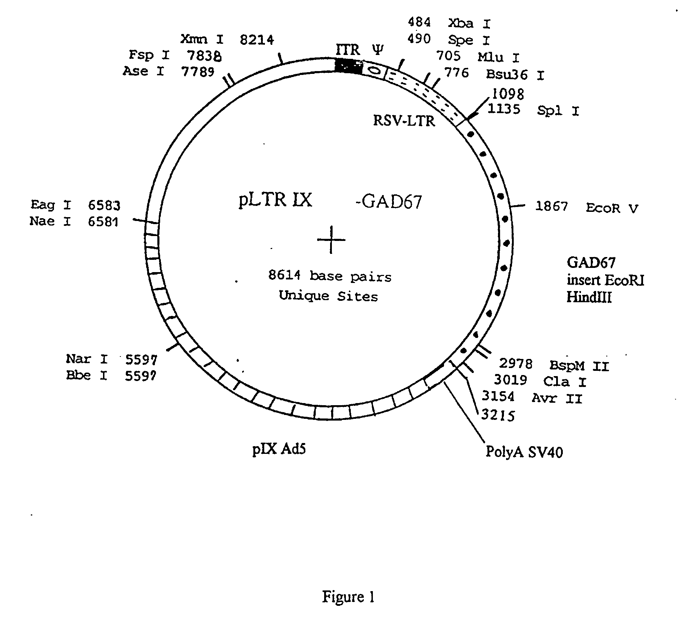

### Exemple 1. Construction du vecteur pLTR IX GAD67 portant le gène codant pour la GAD67 sous le contrôle du promoteur du LTR du virus du sarcome de rous (figure 1).

Cet exemple décrit la construction d'un vecteur comprenant une séquence d'ADNc codant pour la GAD67, sous le contrôle d'un promoteur constitué par le LTR du virus du sarcome de rous (LTR-RSV).

1.1. Vecteur de départ (pLTR IX) : Le vecteur pLTR IX contient en particulier la région gauche de l'adénovirus Ad5 comprenant l'ITR et le site d'encapsidation, le promoteur LTR de RSV, et une région de l'adénovirus Ad5 allant du gène pIX jusqu'au site de restriction EagI, permettant la recombinaison homologue in vivo. Ce vecteur a été décrit par Stratford-Perricaudet et al. (J. Clin. Invest. 90 (1992) 626).

1.2. Construction d'une séquence ADNc codant pour la GAD67.

Pour permettre la réalisation de vecteurs selon l'invention, une séquence d'ADNc codant pour la GAD67 de rat a été construite comme suit :
- Un clone d'ADNc correspondant à l'ARNm de la glutamate décarboxylase GAD67 a été isolé par criblage immunologique d'une banque d'expression d'ADNc de cerveau de rat construite dans le vecteur lambda GT11 (Julien et al., Neurosci. Letters 73 (1987) 173). La nature et la position exacte de la séquence codant pour la GAD67 dans le clone isolé ont été déterminées par séquençage de l'insert. L'insert contenu dans ce clone a ensuite été isolé par digestion au moyen de l'enzyme EcoRI, puis sous-cloné au site correspondant du vecteur pSPT18 ou Bluescript (Pharmacia).

1.3. Construction du vecteur pLTR IX GAD67

Cet exemple décrit la construction du vecteur pLTR IX GAD67 contenant la séquence codant pour la GAD67 sous contrôle du LTR du virus RSV, ainsi que des séquences de l'adénovirus Ad5 permettant la recombinaison in vivo.

Le fragment EcoRI-HindIII de 2 kb a été isolé par digestion enzymatique à partir de la construction préparée dans l'exemple 1.2. Ce fragment de 2 kb contient la séquence codante de la GAD67, et s'étend aux 118 pb en amont du codon d'initiation de la traduction, et aux 70 pb situées en aval du codon de terminaison. Ce fragment a été isolé et purifié par électrophorèse sur un gel d'agarose LMP ("Low Melting Point"), puis traité par l'ADN polymérase de T4 pour obtenir des extrémités franches. Ce fragment a ensuite été inséré au site compatible Sall du vecteur pLTR IX (exemple 1.1.) pour générer le vecteur pLTR IX GAD67 (figure 1). L'ensemble de la séquence nucléotidique de l'insert GAD67 a ensuite été vérifiée par séquençage didéoxynucléotides.

### Exemple 2. Fonctionalité du vecteur pLTR IX GAD67

La capacité du vecteur pLTR IX GAD67 à exprimer sur culture cellulaire une forme biologiquement active de la GAD a été démontrée par transfection transitoire de cellules 293. Pour cela, les cellules (2.10⁶ cellules par boite de 10 cm de diamètre) ont été transfectées (8 µg de vecteur) en présence de Transfectam. L'expression de la séquence codant pour la GAD et la production d'une protéine biologiquement active sont mises en évidence par les tests suivants :
- Détection et quantification des ARNm GAD par la technique de Northern blot (voir par exemple Julien et al., Neurosci. Letters 73 (1987) 173 pour un protocole détaillé)
- Révélation de la protéine GAD par la technique de Western blot en utilisant l'anticorps polyclonal K2 (Chemicon) (voir également Julien et al., Neurosci. Letters 73 (1987) 173 pour un protocole détaillé)
- Mise en évidence et quantification de l'activité GAD (synthèse de GABA) à partir du lysat cellulaire. Pour cela, 48 heures après la transfection, les cellules sont lysées et le lysat est clarifié par centrifugation à 100 000 g. Le lysat est alors incubé en présence d'un anticorps anti-GAD, lui-même fixé sur des billes de sépharose. L'anticorps utilisé est en particulier l'anticorps #1440, qui présente la propriété de préserver l'activité catalytique de la GAD (Oertel et al., Neurosci. 6 (1981) 2715). Il est entendu que d'autres anticorps présentant des propriétés comparables peuvent être utilisés et éventuellement préparés. Le complexe anticorps-GAD active est ensuite séparé du lysat cellulaiore et incubé en présence de [1-¹⁴C] L-glutamate. Le ¹⁴CO2 libéré au cours de la réaction, qui reflète la transformation du glutamate en GABA, est piégé sur un filtre imbibé de hyamine et compté en scintillation liquide. Deux controles négatifs permettent de soustraire le bruit de fond et ainsi de déterminer le signal spécifique : un antisérum pré-immun et un inhibiteur spécifique de la GAD, le γ-acétylénique-GABA (voir aussi Julien et al., Neurosci. Letter 73 (1987) 173).

### Exemple 3. Construction d'un adénovirus recombinant Ad-GAD67 contenant une séquence codant pour la GAD67

Le vecteur pLTR IX GAD67 a été linéarisé et cotransfecté avec un vecteur adénoviral déficient, dans les cellules helper (lignée 293) apportant en *trans* les fonctions codées par les régions E1 (EIA et E1B) d'adénovirus.

Plus précisément, l'adénovirus Ad-GAD67 a été obtenu par recombinaison homologue in vivo entre l'adénovirus mutant Ad.d11324 (Thimmappaya et al., Cell 31 (1982) 543) et le vecteur pLTR IX GAD67, selon le protocole suivant : le plasmide pLTR IX GAD67 et l'adénovirus Ad-d11324, linéarisé par l'enzyme ClaI, ont été co-transfectés dans la lignée 293 en présence de phosphate de calcium, pour permettre la recombinaison homologue. Les adénovirus recombinants ainsi générés ont été sélectionnés par purification sur plaque. Après isolement, l'ADN de l'adénovirus recombinant a été amplifié dans la lignée cellulaire 293, ce qui conduit à un surnageant de culture contenant l'adénovirus défectif recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'adénovirus Ad-GAD67 peut être conservé à -80°C dans 20 % de glycérol.

### Exemple 4. Fonctionalité de l'adénovirus Ad-GAD67

La capacité de l'adénovirus Ad-GAD67 à infecter des cellules en culture et à exprimer dans le milieu de culture une forme biologiquement active de la GAD67 a été démontrée par infection des lignées 293 humaine. La présence de GAD67 actif dans le surnageant de culture a ensuite été déterminée dans les mêmes conditions que dans l'exemple 2.

Ces études permettent de montrer que l'adénovirus exprime bien une forme biologiquement active de la GAD67 en culture cellulaire.

### Exemple 5 : Transfert in vivo du gène GAD67 par un adénovirus recombinant

Cet exemple décrit le transfert du gène GAD67 in vivo au moyen d'un vecteur adénoviral selon l'invention. Il montre comment les effets des vecteurs de l'invention sur différents modèles animal peuvent être mis en évidence.

L'adénovirus recombinant GAD67 peut être injecté en différents points du sysème nerveux. En particulier, l'injection est réalisée dans les différents points suivants : dans le noyau médian du septum, dans la partie dorsale de l'hippocampe, dans le striatum, dans la substance noire et dans le cortex entorhinal.

L'adénovirus injecté est l'adénovirus Ad-GAD67 préparé dans l'exemple 3, utilisé sous forme purifiée (3,5 10⁶ pfu/µl), dans une solution saline phosphate (PBS). Les injections sont réalisées à l'aide d'une canule (diamètre extérieur 280 µm) connectée à une pompe. La vitesse d'injection est fixée à 0,5 µl/min, après quoi, la canule reste en place pendant 4 minutes supplémentaires avant d'être remontée.

Les volumes d'injection dans l'hippocampe, le septum, le striatum et la substance noire sont respectivement 3, 2, 2x3 et 2 µl. La concentration d'adénovirus injectée est de 3,5. 10⁶ pfu/µl.

Pour l'injection dans l'hippocampe, les coordonnées stéréotaxiques sont les suivantes : AP=-4; ML=3,5; V=-3,1 (les coordonnées AP et ML sont déterminées par rapport au bregma, la coordonnée V par rapport à la surface de l'os crânien au niveau du bregma.

Pour l'injection dans le septum, les coordonnées stéréotaxiques sont les suivantes : AP=1; ML=1; V=-6 (les coordonnées AP et ML sont déterminées par rapport au bregma, la coordonnée V par rapport à la surface de l'os crânien au niveau du bregma. Dans cette condition, la canule présente un angle de 9 degrés par rapport à la verticale (dans le sens médio-latéral) afin d'éviter le sinus veineux médian.

Pour l'injection dans la substance noire, les coordonnées stéréotaxiques sont les suivantes : AP=-5,8; ML=+2; V=-7,5 (les coordonnées AP et ML sont déterminées par rapport au bregma, la coordonnée V par rapport à la dure-mère).

Pour les injections dans le striatum, les coordonnées stéréotaxiques sont les suivantes : AP=+0,5 et -0,5; ML=3; V=-5,5 (les coordonnées AP et ML sont déterminées par rapport au bregma, la coordonnée V par rapport à la dure-mère).

Les effets thérapeutiques de l'administration de l'adénovirus selon l'invention sont mis en évidence par trois types d'analyse : une analyse histologique et immunohistochimique, une analyse quantitative et une analyse comportementale, qui témoignent de la capacité des vecteurs de l'invention à produire in vivo une GAD biologiquement active.

### Exemple 6. Construction du plasmide rétroviral pMoMuLV-GAD portant le gène codant pour la GAD 67

L'ADNc de la glutamate décarboxylase de rat qui a été inséré dans un plasmide rétroviral comprend 163 paires de bases non codantes en 5' et 377 en 3'. La phase ouverte de lecture codant pour la GAD comporte 1782 paires de bases. Cet ADNc a été inséré entre les deux LTR du vecteur, sous le contrôle transcriptionnel du LTR 5'. La séquence rétrovirale contient également une séquence y qui permet son encapsidation. Enfin, le plasmide contient un gène de résistance à l'ampicilline qui permet de sélectionner les bactéries après leur transformation. Schéma de la séquence rétrovirale recombinante du plasmide:

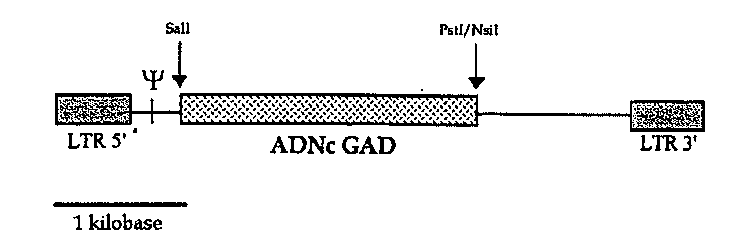
SalI
PstI/NsiI
Ψ
LTR 5'
ADNc GAD
LTR 3'
1 kilobase

Cet ADNc de la GAD 67 de rat a été extrait du plasmide pY21-GAD par coupure SalI/PstI pour générer un fragment de 2,32 kilobases comprenant la séquence codant pour la GAD. 28 mg du plasmide pY21-GAD ont été coupés avec 5 unités de chaque enzyme pendant une heure à 37°C. Après digestion, l'ADN a été précipité à l'éthanol puis l'insert GAD a été purifié après électrophorèse en gel d'agarose 1% à température de fusion basse.

Le vecteur pMoMuLV-TH qui comprend les séquences rétrovirales nécessaires à la transcription et à l' intégration (respectivement "LTR" et "Ψ") a été coupé par les enzymes Sall et NsiI. Cette coupure a permis de retirer l'ADNc de la tyrosine hydroxylase du vecteur et de générer un site de clonage pour l'ADNc de la GAD (les enzymes NsiI et PstI forment des extrémités cohésives compatibles). 10 mg d'ADN ont été coupés avec 5 unités d'enzymes pendant une heure à 37°C. Le vecteur a été purifié par la même méthode que l'ADNc de la GAD.

90 ng de l'ADNc de la GAD et 360 ng du vecteur pMoMuLV ont été ligaturés sur la nuit à 16°C en présence de 4 unités d'ADN Ligase du bactériophage T4.

La moitié de l'ADN ligaturé a été introduit dans des bactéries compétentes *E.Coli* (XL1 Blue) par électroporation et les cellules transformées ont été sélectionnées sur boîte de Petri LB/agarose contenant 100 mg/ml d'ampicilline. Les clones recombinant pMoMuLV-GAD ont été identifiés par digestion par les enzymes HindIII et KpnI qui coupent de façon assymétrique le vecteur et l'insert. L'analyse de 24 clones bactériens par coupure HindIII/KpnI a permis d'en isoler 4, correspondant au plasmide attendu. Un de ces clones a été utilisé pour la production du plasmide rétroviral.

Le plasmide pMoMuLV-GAD a été préparé par purification de l'ADN plasmidique d'un clone recombinant sur gradient CsCl selon la méthode de Birnboim et Doly *(Nucleic Acids Research,* Vol 7: 1513-1523, 1979).

### Exemple 7. Transfection des cellules productrices de rétrovirus.

Pour cette étape, nous avons utilisé la lignée cellulaire Ψ-2 qui possède un provirus exprimant toutes les fonctions rétrovirales requises pour l'encapsidation de transcrits rétroviraux mais qui est défective pour le signal d'encapsidation Ψ. A cause de cette délétion, les transcrits du provirus ne sont pas encapsidés. Par contre, un rétrovirus défectif pour les fonctions d'encapsidation mais possédant une séquence y pourra être encapsidé par cette cellule trans-complémentante.

Les cellules Ψ-2 ont été cotransfectées avec le plasmide pMoMuLV-GAD et un plasmide contenant un gène de résistance à la néomycine (pUC-SVNEO) à l'aide d'un précipité de phosphate de calcium (Chen et Okayama, *Molecular and Cellular Biology,* Vol 7:2745-2752, 1988). Un précipité comprenant 20 mg de pMoMuLV-GAD et 2 mg de pUC-SVNEO a été laissé en contact pendant 18 heures avec 10⁶ cellules. Ensuite, la culture a été poursuivie en milieu DMEM (Gibco) supplémenté en antibiotiques (penicilline: 100 unités/ml; streptomycine: 100 mg/ml) et en sérum de veau foetal (10% vol/vol).

Deux jours après transfection, les cellules sont passées sur sept boîtes de Pétri (10 cm de diamètre) et cultivées dans le même milieu additionné de 600 mg/ml de néomycine afin de sélectionner les cellules transfectées.

Quinze jours après le début de la sélection, les clones résistants à la néomycine sont prélevés à la pipette pasteur et cultivés en flacon de 25 cm².

Lorsque les cellules des clones Ψ-2 résistants à la néomycine sont confluentes, elles sont mises en contact avec du milieu neuf pendant douze heures. Ce milieu, qui contient les rétrovirus produits par les cellules Ψ-2, est récolté, congelé dans l'azote liquide et stocké à -80°C jusqu'à utilisation.

### Exemple 8. Criblage des clones Ψ-2 par infection de cellules de la lignée NIH-3T3.

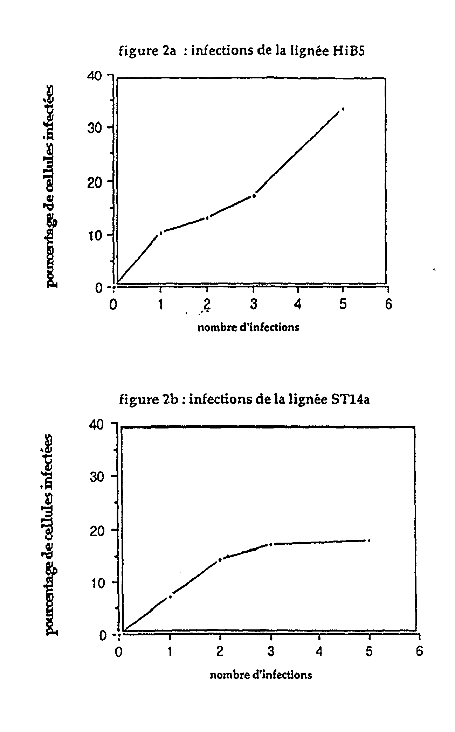

Afin de déterminer la qualité et la quantité des rétrovirus produits par les différents clones Ψ-2, ces derniers ont été testés indirectement en étudiant leur potentiel infectieux sur des cellules de la lignée NIH-3T3 (lignée cellulaire de type fibroblastique, immortalisée à partir de fibroblastes de souris). Pour cela, les surnageants rétroviraux des 35 clones Ψ-2 ont été utilisés pour infecter les cellules, et, après infection, elles ont été soit récoltées pour effectuer un western blot, soit fixées pour détecter la GAD par immunocytochimie (figure 2). Les résultats de ces expériences nous ont permis de sélectionner le clone Ψ-2 présentant le potentiel infectieux le plus fort.

### Exemple 9. Infection de lignées cellulaires HiB5 et ST14a par les rétrovirus

Les surnageants rétroviraux provenant du clone Ψ-2, sélectionné dans l'exemple 8, ont été utilisés pour infecter deux lignées cellulaires progénitrices du système nerveux central HiB5 et STl4a :
- HiB5: lignée cellulaire dérivant d'une culture primaire d'hippocampe de rat (16^{ème} jour de la vie embryonnaire) immortalisée par l'antigène T thermosensible de SV40. Les cellules de cette lignée sont des progéniteurs de cellules de types neuronales et gliales (Renfranz *et al., Cell* Vol 66: 713-729, 1991),
- ST14a: cette lignée a été immortalisée de la même façon que la lignée précédente mais elle provient d'une culture primaire de striatum de rat.

Les cellules des lignées HiB5 et ST14a ont la propriété de se diviser in vitro à 33°C mais arrêtent de se multiplier une fois transplantées dans le système nerveux central des rongeurs dont la température corporelle est de 39°C. Ces cellules peuvent donc se cultiver facilement *in vitro* (à 33°C) et ne sont pas tumorigènes *in vivo.*

Elles ne doivent pas être confluentes lors de l'infection : la division cellulaire (et la synthèse d'ADN qu'elle entraîne) est nécessaire pour l'intégration du génome rétroviral dans le génome de la cellule hôte (Lo *et al., Molecular Neurobiology* Vol 2:156-182, 1988).

Le milieu est changé pour du surnageant rétroviral auquel a été ajouté 10 mg/mI de polybrène. Les cellules sont laissées au contact du surnageant rétroviral pendant douze heures, puis le milieu est changé pour du milieu normal.

Ces cellules ne s'infectant pas facilement, nous avons eu recours à une méthode d'infections répétées : les cellules ont été infectées cinq fois à raison d'une fois par jour pendant cinq jours. Le pourcentage de cellules infectées a été déterminé par immunocytochimie après chaque infection.

Après cinq infections, le pourcentage de cellules infectées était de 33% pour la lignée HiB5 et de 17% pour la lignée ST14a (figures 2a et 2b).

Nous avons entrepris le sous clonage de ces lignées cellulaires infectées afin d'obtenir des clones cellulaires exprimant la GAD. Des boîtes de Pétri (diamètre de 10 cm) ont été ensemencées avec les cellules infectées à raison de 1000, 500 ou 200 cellules par boîte. Les clones cellulaires isolés ont été prélevés après 15 jours de culture. Nous avons isolé 17 clones provenant de la lignée HiB5 et 20 de la lignée ST14a.

### Exemple 10. Fonctionnalité du rétrovirus pMoMuLV-G

L'activité enzymatique de la GAD est évaluée par la méthode de Hamel *et al. (Journal of Neurochemistry* Vol 39:842-849, 1982) modifiée par Brass *et al. (Journal of Neurochemistry* Vol 59:415-424, 1992). Le principe de cette mesure d'activité enzymatique repose sur la détection du GABA formé à partir d'un précurseur tritié (³H-glutamate) en incubant à 37°C l'extrait protéique contenant la GAD. Cette réaction se fait en présence du cofacteur de la GAD (phosphate de pyridoxal) et d'un inhibiteur de la GABA transaminase afin de préserver le GABA formé. Le GABA tritié formé est ensuite séparé du glutamate grâce à une colonne de résine échangeuse d'ions puis la quantité de GABA formé est évaluée à l'aide d'un compteur à scintillation. Le protocole détaillé du dosage est le suivant:
Les cellules sont lysées dans un tampon Na phosphate (pH=7,4) contenant de l'EDTA (10 mM), de la sérum albumine de boeuf (SAB, 5 mg/l), et du Triton X-100 (0,5% vol/vol).
La réaction enzymatique est effectuée à partir de 80 ml de lysat cellulaire auxquels sont ajoutés: (1) 10 ml d'une solution contenant du phosphate de pyridoxal (200 mM, cofacteur de la GAD) et de l'AminoEthyl-isoThiouronium bromide (10 mM, inhibiteur de la GABA transaminase); (2) 10 ml d'une solution contenant 5 mM de glutamate non marqué et 10⁶ dpm de glutamate tritié purifié extemporanément selon la méthode de Bird (1976).
Le mélange réactionnel est ensuite incubé 90 minutes à 37°C. La réaction est stoppée par addition de 0,5 ml d'une solution cuprique préparée extemporanément (CuCl₂ 6,92 g/l; Na₃PO₄ 59,6 g/l; borate de sodium 21,68 g/l) qui permet de complexer le glutamate. La suspension est gardée sur la glace pendant 10 minutes afin que le complexe se forme, puis le précipité de cuivre est éliminé par centrifugation (13 000 rpm, 5 minutes).
Le surnageant est déposé sur une colonne de résine Dowex (AG-1X8 acetate, Biorad) qui retient le glutamate restant. La colonne est lavée deux fois avec 0,5 ml d'eau.
Le GABA produit est déterminé par comptage de l'éluat dissout dans 10 ml de liquide de scintillation (Aqueous Counting Scintillant, Amersham).
Le bruit de fond de la réaction est évalué en remplaçant le lysat cellulaire par du tampon de lyse sans cellules. La concentration des protéines du lysat cellulaire est déterminée suivant la méthode de Bradford *(Analogical Biochemistry* Vol 72:248-254, 1976). L'activité enzymatique est exprimée en nmoles de GABA formé/mg protéines/heure.

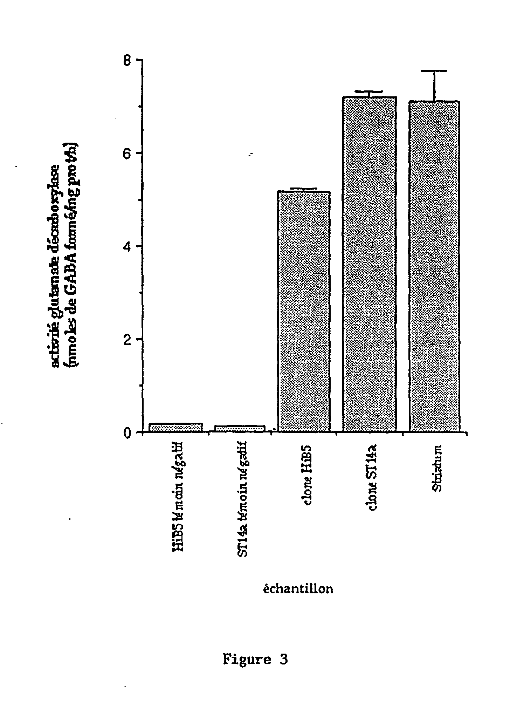

La mesure de l'activité glutamate décarboxylase a été effectuée sur chaque clone provenant des lignées infectées par le rétrovirus recombinant codant pour la GAD. En parallèle, l'activité GAD a été déterminée sur des extraits de striatum et du clone y-2 producteur de rétrovirus. Les résultats du dosage sont présentés en figure 3. Ainsi, sur les 17 clones issus des cellules HiB5 infectées, 3 exprimaient des forts taux de GAD. De même, pour les clones provenant de la lignée ST14a, 3 sur 20 exprimaient fortement la GAD. Un clone HiB5 et un clone STl4a ont été retenus pour la transplantation, leur activité enzymatique glutamate décarboxylase atteignant respectivement:
- clone HiB5: 5,17 nmoles/mg prot/heure.
- clone ST14a:7,20 nmoles/mg prot/heure.
- striatum: 7,13 nmoles/mg prot/heure.

On peut également procéder à un Western Blot selon le protocole décrit Towbin *et al.* (*P.N.A.S.* Vol 76:4350-4354, 1979) :
30 ml de lysat cellulaire (même tampon que pour l'activité cellulaire) est dénaturé au bain-marie bouillant en présence de 15 ml de "bleu Laemli" (Laemmli, *Nature* Vol 227:680-685, 1970). Cette solution est déposée sur gel de polyacrylamide à 10% contenant 0,1% de SDS. La migration se fait sur la nuit à 30 mV. Après migration, les protéines sont transférées sur une membrane de nitrocellulose. La GAD est mise en évidence par immunorévélation.

Les immunorévélations nous ont permis de caractériser la GAD sur les ' membranes de western blot, sur les lignées cellulaires en culture, et sur les coupes de tissus après transplantation. Elles ont été réalisées à partir d'un sérum de lapin immunisé par la GAD. Le sérum a été utilisé dilué au 3000^{e} et incubé sur la nuit à 4°C. L'anticorps primaire a été révélé par la peroxydase (Vectastain ABC kit, Vector Laboratories). Dans le cas des coupes de tissus obtenues après transplantations, l'anticorps primaire a également été révélé par un anticorps secondaire marqué à la fluorescéine (FITC goat anti-rabbit, Nordic)

### Exemple 11. Transfert in vivo du gène GAD67 par un rétrovirus recombinant

Les clones provenant des lignées cellulaires Hib5 et ST14a et montrant une activité enzymatique ont été transplantés dans le striatum de rats de la souche Sprague-Dawley auparavant anesthésiés par l'équitésine.

7,5.105 cellules reprises dans 3 ml de DMEM sans sérum ont été injectées en 3 minutes par stéréotaxie aux coordonnées suivantes:
- antériorité: +0,6 mm par rapport au bregma,
- latéralité: +3 mm par rapport au bregma,
- profondeur: -4,5 mm par rapport à la duremère.

L'aiguille a été laissée en place 3 minutes avant le retrait.

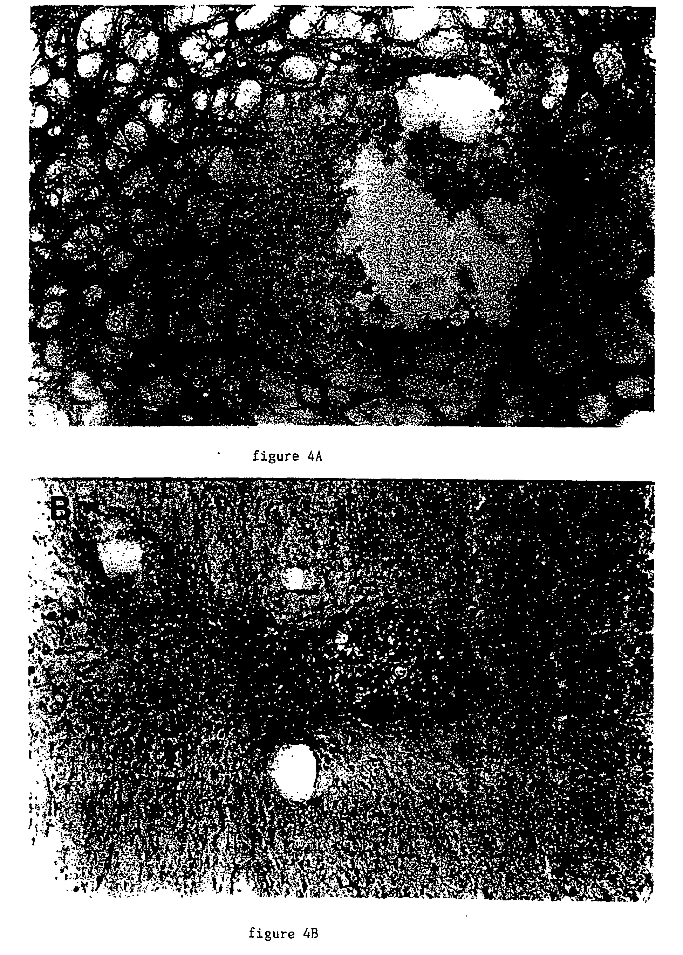

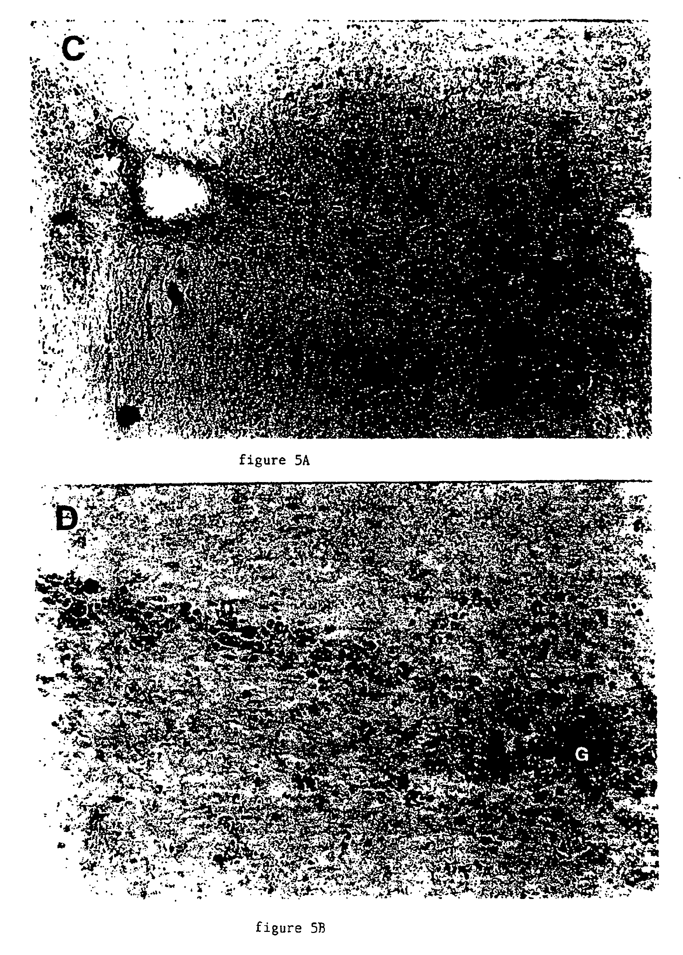

Une à deux semaines après transplantation, les animaux ont été perfusés avec une solution de paraformaldéhyde à 4% maintenue à 4°C et les cerveaux ont été disséqués et sectionnés au microtome à congélation (épaisseur de 40 mm). Une immunohistochimie a été réalisée. L'anticorps primaire (anticorps polyclonal de lapin anti-GAD) a été révélé soit par un anticorps secondaire couplé à la péroxydase, soit par un anticorps secondaire fluorescent. Nous avons ainsi pu mettre en évidence un immunomarquage spécifique de la GAD dans les greffes de cellules HiB5 et ST14a infectées par rapport aux greffons provenant des cellules non infectées (figures 4 et 5) en figures 4A sont représentés les cellules ST14a non infectées, en 4B figure clone HiB5 exprimant le transgène, en 5A et 5B le clone ST14a exprimant le transgène. Dans l'ensemble des figures 4 et 5, des flèches indiquent les cellules infectées exprimant le transgène. Les résultats démontrent que les clones infectés conservent une forte expression du transgène après transplantation.

### EXEMPLE 12 . LIBERATION DE GABA IN VITRO

La libération de GABA par les clones HiB5-GAD et ST14a-GAD retenus pour la transplantion a été étudiée in vitro.

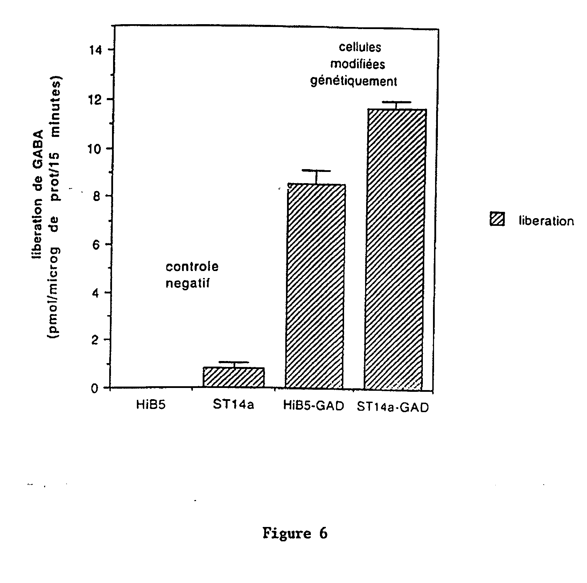

Pour cela, les clones ont été cultivés en plaque de 6 puits, puis rincés avec une solution de type "Hank's balanced salts" (HBSS, Gibco) avant d'être incubés pendant quinze minutes dans le même milieu. Après incubation, le milieu a été récolté, centrifugé pour éliminer les cellules, et analysé par HPLC. Les cellules ont également été récoltées pour évaluer la concentration en protéines. L'HPLC a été réalisée selon la méthode décrite par Kehr et Ungerstedt (J. Neurochem. Vo151:1308-1310, 1988) et à permis de mettre en évidence une libération importante de GABA par les cellules dans leur milieu de culture. Les résultats sont représentés en figure 6.

## Revendications

1. Virus recombinant défectif comprenant une séquence d'ADN codant pour une protéine ayant une activité glutamate décarboxylase (GAD).

2. Virus selon la revendication 1 **caractérisé en ce que** la séquence d'ADN est une séquence d'ADNc.

3. Virus selon la revendication 1 **caractérisé en ce que** la séquence d'ADN est une séquence d'ADNg.

4. Virus selon l'une des revendications 1 à 3 **caractérisé en ce que** la séquence d'ADN code pour tout ou partie de la protéine GAD67 ou d'un dérivé de celle-ci.

5. Virus selon l'une des revendications 1 à 3 **caractérisé en ce que** la séquence d'ADN code pour tout ou partie de la protéine GAD65 ou d'un dérivé de celle-ci.

6. Virus selon l'une des revendications 1 à 5 **caractérisé en ce que** la séquence d'ADN est placée sous le contrôle de signaux permettant son expression dans les cellules nerveuses.

7. Virus selon la revendication 6 **caractérisé en ce que** les signaux d'expression sont choisis parmi les promoteurs viraux.

8. Virus selon la revendication 7 **caractérisé en ce que** les signaux d'expression sont choisis parmi les promoteurs E1A, MLP, CMV et LTR-RSV.

9. Virus recombinant défectif comprenant une séquence d'ADNc codant pour une protéine ayant une activité glutamate décarboxylase (GAD) sous le contrôle du promoteur LTR-RSV.

10. Virus recombinant défectif comprenant une séquence d'ADNg codant pour une protéine ayant une activité glutamate décarboxylase (GAD) sous le contrôle du promoteur LTR-RSV.

11. Virus recombinant défectif comprenant une séquence d'ADN codant pour une protéine ayant une activité glutamate décarboxylase (GAD) sous le contrôle d'un promoteur permettant une expression majoritaire dans les cellules nerveuses.

12. Virus selon l'une des revendications 1 à 11 **caractérisé en ce qu'**il est dépourvu des régions de son génome qui sont nécessaires à sa réplication dans la cellule cible.

13. Virus selon l'une des revendications 1 à 12 **caractérisé en ce qu'**il s'agit d'un adénovirus.

14. Virus selon la revendication 13 **caractérisé en ce qu'**il sagit d'un adénovirus humain de type Ad 2 ou Ad 5 ou canin de type CAV-2.

15. Virus selon l'une des revendications 1 à 12 **caractérisé en ce qu'**il s'agit d'un virus adéno-associé.

16. Virus selon l'une des revendications 1 à 12 **caractérisé en ce qu'**il s'agit d'un rétrovirus.

17. Virus selon la revendication 16 **caractérisé en ce qu'**il s'agit d'un rétrovirus de la famille MoMuLV.

18. Virus selon l'une des revendications 1 à 12 **caractérisé en ce qu'**il s'agit d'un virus de l'herpès (HSV).

19. Utilisation d'un virus selon l'une des revendications 1 à 18 pour la préparation d'une composition pharmaceutique destinée au traitement et/ou à la prévention des maladies neurodégénératives.

20. Utilisation selon la revendication 19 pour la préparation d'une composition pharmaceutique destinée au traitement et/ou à la prévention de la maladie de Parkinson, d'Alzheimer, de Huntington de l'épilepsie ou de l'ALS.

21. Composition pharmaceutique comprenant un ou plusieurs virus recombinants défectifs selon l'une des revendications 1 à 18.

22. Composition pharmaceutique selon la revendication 21 **caractérisée en ce qu'**elle est sous forme injectable.

23. Composition pharmaceutique selon la revendication 21 ou 22 **caractérisée en ce qu'**elle comprend entre 10⁴ et 10¹⁴ pfu/ml, et de préférence 10⁶ à 10¹⁰ pfu/ml adénovirus recombinants défectifs.

24. Cellule de mammifère infectée par un ou plusieurs virus recombinants défectifs selon l'une des revendications 1 à 18.

25. Cellule selon la revendication 24 **caractérisée en ce qu'**il s'agit d'une cellule humaine.

26. Cellule selon la revendication 27 **caractérisée en ce qu'**il s'agit d'une cellule humaine de type fibroblaste, myoblaste, hépatocyte, cellule endothéliale, cellule gliale ou kératynocyte.

27. Implant comprenant des cellules infectées selon les revendications 24 à 26 et une matrice extracellulaire.

28. Implant selon la revendication 27 **caractérisé en ce que** la matrice extracellulaire comprend un composé gélifiant choisi de préférence parmi le collagène, la gélatine, les glucosaminoglycans, la fibronectine et les lectines.

29. Implant selon les revendications 27 ou 28 **caractérisé en ce que** la matrice extracellulaire comprend également un support permettant l'ancrage des cellules infectées.

30. Implant selon la revendication 29 **caractérisé en ce que** le support est constitué préférentiellement par des fibres de polytétrafluoroéthylène.
